(19) Europäisches Patentamt
European Patent Office
Office européen des brevets

(11) Veröffentlichungsnummer: **0 106 018**
**B1**

(12) # EUROPÄISCHE PATENTSCHRIFT

(45) Veröffentlichungstag der Patentschrift: **23.10.85**

(51) Int. Cl.⁴: **C 07 C 51/367**, C 07 C 59/125, C 07 C 149/20

(21) Anmeldenummer: **83105978.7**

(22) Anmeldetag: **18.06.83**

(54) **Verfahren zur Herstellung von carboxymethylierten Alkoholen, Etheralkoholen, Thioalkoholen oder Alkylphenolen.**

(30) Priorität: **18.08.82 DE 3230677**

(43) Veröffentlichungstag der Anmeldung: **25.04.84 Patentblatt 84/17**

(45) Bekanntmachung des Hinweises auf die Patenterteilung: **23.10.85 Patentblatt 85/43**

(84) Benannte Vertragsstaaten: **AT BE CH DE FR GB IT LI NL SE**

(56) Entgegenhaltungen:
**DE - A - 2 759 169**
**DE - B - 2 418 444**
**DE - C - 975 850**
**GB - A - 1 453 009**

(73) Patentinhaber: **CHEMISCHE WERKE HÜLS AG, - RSP Patente / PB 15 - Postfach 13 20, D-4370 Marl 1 (DE)**

(72) Erfinder: **Springmann, Hermann, Dr., Am Mühlenberg 2, D-4358 Haltern (DE)**
Erfinder: **Kosswig, Kurt, Dr., Hoechster Strasse 10, D-4370 Marl (DE)**

**EP 0 106 018 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein verbessertes Verfahren zur Herstellung von carboxymethylierten Alkoholen oder Phenolen und insbesondere von Polyetheralkoholen, das die quantitative oder nahezu quantitative Umsetzung der für die Carboxymethylierungsreaktion eingesetzten Chloressigsäure zu den entsprechenden Carboxylaten in einer technisch leicht durchführbaren Art und Weise ermöglicht. Bei den carboxymethylierten Alkoholen und Etheralkoholen handelt es sich um Verbindungen des Typs

$$R(OCH_2CH_2)_n-O-CH_2COOH,$$

welche bei entsprechender Wahl des Restes R und des Oxethylierungsgrades n wertvolle Tenside darstellen, die z. B. bei der tert. Erdölförderung eingesetzt werden können (Tenside Detergents 16 (1979), 256—261).

Eine bekannte Methode zur Herstellung von Ethercarbonsäuren bzw. Polyethercarbonsäuren besteht darin, daß man aus Alkohol bzw. Etheralkohol und Alkalihydroxid zunächst die entsprechenden Alkoholate herstellt und diese anschließend mit Natriumchloracetat carboxymethyliert (DE-PS 975 850). Eine wesentliche Verbesserung dieses Verfahrens wird in der neueren Literatur angegeben. Gemäß DE-PS 2 418 444 wird der Polyetheralkohol mit dem Natriumchloracetat vermischt und zu dieser Mischung die äquivalente Menge Natriumhydroxid entweder in Form von Pulver oder als wäßrige, ca. 50%ige Lösung zugegeben, wobei im letzteren Fall das eingebrachte Wasser gleichzeitig möglichst weitgehend aus dem Reaktionsgemisch entfernt wird (vgl. Beispiel 5).

Eine sehr ähnliche Arbeitsweise entnimmt man der japanischen Patentanmeldung Sho-50-24 215 (wir beziehen uns auf den Text einer uns vorliegenden englischen Übersetzung von Esaki Patent Office, Tokio) der Firma Kao Sekken K. K.

In beiden Literaturstellen wird der erreichbare Umsetzungsgrad zu den entsprechend carboxymethylierten Verbindungen übereinstimmend mit etwa 90 Mol-% angegeben, wenn mit einem Molverhältnis von Oxethylat zu Chloracetat wie 1 : 1 gearbeitet wird (Vgl. DE-PS 2 418 444, Beispiel 5, und Sho-50-24 215, Beispiele 1 und 2). Werden noch höhere Umsetzungsgrade verlangt, so ist dies nur zu erreichen, wenn ein erheblicher Überschuß (20 bis 50%) des Chloracetats gegenüber dem Alkohol bzw. Etheralkohol eingesetzt wird (siehe DE-PS 2 418 444, Spalte 2, Zeilen 31 bis 36). In Beispiel 11 dieser Patentschrift werden auf 1,5 Mol Oxethylat insgesamt 2,25 Mol Natriumchloracetat eingesetzt, um einen Umsetzungsgrad von 98,6% zu der erwünschten Carbonsäure zu erreichen. Nachteilig erscheint außerdem die lange Reaktionszeit von ca. 36 Stunden, die zur Erreichung dieses Umsetzungsgrades erforderlich ist.

Ziel der vorliegenden Erfindung war es daher, das Verfahren der Carboxymethylierung so zu gestalten, daß eine möglichst quantitative Umsetzung des eingesetzten Chloracetats zu dem erwünschten carboxymethylierten Produkt erreicht wird. Darüber hinaus sollte das Verfahren insgesamt wirtschaftlicher arbeiten, etwa durch den Einsatz von leicht dosierbaren Komponenten in flüssiger Form, so daß eine großtechnischen Ausführung des Verfahrens ermöglicht wird.

Der Einsatz von wäßriger Natronlauge ist bereits Stand der Technik (DE-PS 2 418 444, Beispiele 5 und 6, sowie JP-PS Sho-50-242 415). Die Verwendung einer wäßrigen Chloressigsäurelösung ist jedoch nicht bekannt.

Zwar findet sich in der DE-PS 2 418 444 der Hinweis, man könne anstelle des Natriumchloracetats auch die freie Säure einsetzen; es wurden jedoch keine näheren Hinweise auf die Durchführung dieses Verfahrens gegeben. Von den im Handel zur Verfügung stehenden Lieferformen ist das Natriumchloracetat die wertvollste und auch die am schwierigsten zu handhabende Lieferform, die freie Chloressigsäure ist weniger wertvoll, muß jedoch in geschmolzener Form dosiert werden, was einen erheblichen Aufwand für beheizbare Behälter, Pumpen und Rohrleitungen erfordert.

Die am wenigsten wertvolle Lieferform des Handels ist eine 80%ige wäßrige Lösung der Chloressigsäure, die ohne Aufwand gefördert und dosiert werden kann. Aus wirtschaftlichen und technischen Gründen ist daher der Einsatz der wäßrigen Chloressigsäure vorzuziehen.

Aus der Literatur ist zu entnehmen, daß nach herrschender Meinung Wasser einen außerordentlich negativen Einfluß auf den Verlauf der Carboxymethylierungsreaktion nimmt. Beispielsweise wird in der älteren Literatur (NL-PS 64 534) vorgeschlagen, das entstehende Reaktionswasser durch Zusatz von Calciumcarbid im Reaktionsgemisch zu binden. Auch in der bereits erwähnten neueren Patentliteratur finden sich Hinweise auf einen negativen Einfluß des Wassers (DE-PS 2 418 444, Spalte 3, Zeilen 14 bis 18). In der japanischen Patentanmeldung (Sho-50-24 215) wird der Einfluß des Wassers ausführlicher diskutiert: Eine Veränderung des Reaktionsdruckes bei gegebener Temperatur beeinflußt den Wassergehalt im System (vgl. Seite 7 der o. a. englischen Übersetzung).

Je mehr der Druck erniedrigt wird, d. h. umso weniger Wasser sich im Reaktionssystem befindet, um so mehr wird die Hauptreaktion (d. h. die erwünschte Reaktion) begünstigt, und je weniger der Druck gesenkt wird, um so mehr Wasser befindet sich im Reaktionssystem und um so weniger wird die Hauptreaktion begünstigt.

Weiter heißt es: Da die Reaktion eine ionische Reaktion ist, wird die Gesamtreaktionsgeschwindigkeit groß, wenn eine geringe Menge Wasser anwesend ist. Als »Gesamtreaktionsgeschwindigkeit« wird an anderer Stelle des Patents die Verbrauchsgeschwindigkeit der Halogencarbonsäure definiert. Wenn also durch die Anwesenheit von Wasser diese erhöht wird, andererseits, die Hauptreaktion, nämlich die Bildung des carboxymethylierten Produktes durch Wasser beeinträchtigt wird, so heißt das, daß die Anwesenheit von Wasser die unerwünschte Hydrolysereaktion der Halogencarbonsäure fördern muß und diese somit für die erwünschte Hauptreaktion verloren geht.

Überraschenderweise wurde nun gefunden, daß bei einem Verfahren zur Herstellung von carboxymethylierten Alkoholen, Etheralkoholen oder Alkylphenolen und deren Oxethylaten, wie es durch die Patentansprüche beschrieben wird, diese Verhältnisse nicht zutreffen. Obwohl eine gewisse Wasserkonzentration eingehalten wird, erleidet die Carboxymethylierungsreaktion keine Beeinträchtigung, wie dies nach dem vorstehenden Ausführungen erwartet werden könnte. Vielmehr reagiert die eingesetzte Chloressigsäure unter Bildung der erwünschten carboxymethylierten Produkte, ohne daß es in einem wesentlichen Umfang infolge von Hydrolyse zur Entstehung von Glykolsäure kommt. Die eingesetzten Chloressigsäure wird bei vollständigem Umsatz mit einer Selektivität von über 90 bis nahezu 100% zu den carboxymethylierten Verbindungen umgesetzt.

Ein sehr wichtiges Element des neuen, erfindungsgemäßen Verfahrens ist daher die Einhaltung einer in einem verhältnismäßig engen Bereich liegenden Konzentration des Wassers in dem Reaktionsgemisch. Es ergeben sich sehr kurze Reaktionszeiten, und die eingesetzte Chloressigsäure wird mit einem Anteil von mehr als 95% für die gewünschte Carboxymethylierungsreaktion ausgenutzt.

Aus diesem Grunde ist es auch möglich, sowohl das Alkalihydroxid als auch die Chloressigsäure in Form von wäßrigen Lösungen einzusetzen, was besondere, aufwendige Dosiereinrichtungen für pulverförmige Stoffe überflüssig macht. Es genügt, in einem geeigneten Rührgefäß den Alkohol oder Etheralkohol vorzulegen und unter Rühren in einem Temperaturbereich von ca. 60 bis 120°C und bei einem Druck von ca. 10 bis 100 mbar die wäßrige Chloressigsäure und die wäßrige Alkalilauge über getrennte Zuleitungen in das Rührgefäß einzugeben. Die Chloressigsäure sollte zweckmäßigerweise als 80%ige wäßrige Lösung (handelsüblich und die Alkalilauge als 50%ige wäßrige Lösung eingesetzt und mittels Dosierpumpen gefördert werden. Hierbei muß die Alkalilauge in doppelt molarer Menge, bezogen auf die Chloressigsäure, zudosiert werden. Alkalilauge und Chloressigsäure werden zwar, gemäß der Anweisung des 1. Anspruchs, gleichzeitig zugegeben, es hat sich aber als zweckmäßig erwiesen, die Zufuhr der Lauge mit einem geringen zeitlichn Vorsprung, nämlich 5 bis 10 Minuten vor der Zufuhr der Chloressigsäure zu beginnen; damit ist während der Reaktionsdauer stets ein geringer Überschuß an Alkali gewährleistet, der gegen Reaktionsende verschwindet. Als Base können eingesetzt werden: eine wäßrige $Ca(OH)_2$-Suspension, Lösungen von NaOH, KOH oder Mischungen dieser Basen.

Die notwendige, optimale Wasserkonzentration im Reaktionsgemisch liegt zwischen 0,30 bis 1,25 Gewichtsprozent, vorzugsweise jedoch im Bereich von 0,3 bis 0,8 Gewichtsprozent. Es ist leicht möglich, durch die Wahl des Wertepaares von Druck und Temperatur einen vorteilhaften Wasserwert einzustellen, wobei ein höherer Druck mit einer höheren Temperatur zu kombinieren ist. Beispielsweise kann man die Reaktion bei einem Druck von 12 mbar und einer Temperatur von 70°C mit demselben Ergebnis durchführen wie bei einem Druck von 25 mbar und 90°C.

Die Zulaufgeschwindigkeit kann innerhalb eines weiten Bereichs gewählt werden (vgl. Beispiele 3 und 3a, Tabelle I), vorausgesetzt, daß die mit den wäßrigen Lösungen eingebrachte Wassermenge so weit aus dem Reaktionsgemisch rasch genug entfernt wird, daß die erfindungsgemäße Wasserkonzentration von 0,3 bis 1,25 Gewichtsprozent eingehalten werden kann.

Die Reaktion wird zweckmäßigerweise in einem zylindrischen Rührgefäß durchgeführt, das mit einem Blattrührer ausgerüstet ist. Das Gefäß ist mit einem Doppelmantel versehen, so daß durch Kühlung oder Heizung die erforderliche Reaktionstemperatur eingehalten werden kann. Im unteren Ablauf des Reaktionsgefäßes wird über ein T-Stück z. B. mittels einer Zahnradpumpe Produkt in einer geringen Menge entnommen und wieder zurück in den oberen Teil des Reaktors gefahren. So besteht die Möglichkeit, während der Durchführung der Reaktion ohne Unterbrechung des Vakuums Proben zu entnehmen und zu analysieren. Beispielsweise kann der Seitenstrom kontinuierlich durch eine Meßstelle geleitet werden, die den Wassergehalt registriert. Die Zufuhr der Lauge und der Chloressigsäure kann aus Tropftrichtern erfolgen, zweckmäßigerweise werden jedoch exakt arbeitende Dosierpumpen eingesetzt.

Die Leitungen für die Zufuhr der Natronlauge einerseits und der Chloressigsäure andererseits sind in dem vorgelegten Oxethylat abgetaucht. Die Hauptmenge des abgezogenen Wassers wird über einen wirksamen Kühler kondensiert und abgetrennt, der Rest — falls notwendig — durch gut gekühlte Kühlfallen abgeschieden. Eventuell mitgehende organische Substanz wird in einem Abscheider getrennt und zurückgeleitet. Die Reaktion ist praktisch zu Ende, wenn die Komponenten Chloressigsäure und Natronlauge vollständig zugegeben sind, was durch den weitgehenden Verbrauch der Natronlauge festgestellt werden kann. Daher genügt eine kurze Nachrührphase von wenigen Minuten.

Die weitere Aufarbeitung des so hergestellten Rohprodukts richtet sich nach dem angestrebten Verwendungszweck. Für bestimmte Einsatzzwecke, z. B. zum Tensidfluten, kann das so erhaltene Rohprodukt eingesetzt werden. Eine Abtrennung des als Nebenprodukt entstandenen Natriumchlorids

ist in diesem Fall nicht notwendig. Ist die Abtrennung jedoch erwünscht, so kann durch Ansäuern der Reaktionsmischung und Erwärmung auf etwa 95°C eine Trennung in eine organische, obere Phase erreicht werden, die die freie Säure enthält und die leicht von der unteren, wäßrigen Phase, die das Kochsalz gelöst enthält, abgetrennt werden kann (vgl. DE-PS 2 418 444, Spalte 3, Zeile 19).

Als Verbindungen, welche im vorliegenden Verfahren carboxymethyliert werden können, kommen in Frage: gesättigte oder ungesättigte, geradkettige oder verzweigte Alkohole mit 8 bis 20 C-Atomen, wie beispielsweise n-Octanol, 2-Ethylhexanol, Caprylalkohol, n-Decanol, Laurylalkohol, Myristylalkohol, Cetylalkohol, Stearylalkohol, Eicosylalkohol, Oleyalkohol, Thioalkohole, wie beispielsweise Laurylmercaptan, Decylmercaptan, Myristylmercaptan, Stearylmercaptan, (Di)-Alkylphenole mit 7 bis 18 C-Atomen in der Alkylkette, wie z. B. Heptiylphenol, Octylphenol, Nonylphenol, Decylphenol, Undecylphenol, Dodecylphenol oder Dinonylphenol, ferner die Oxethylierungsprodukte der genannten Alkohole, Thiolalkohole und Alkylphenole mit 1 bis 30 Ethylenoxidgruppen, vorzugsweise 2 bis 15 Ethylenoxidgruppen.

Ist das gebildete Reaktionsprodukt eine bei der Reaktionstemperatur feste Substanz (wie z. B. bei der Carboxymethylierung von Myristylalkohol, Beispiel 9), so ist die Mischung unter Umständen nicht mehr rührfähig. In diesem Fall kann die zu carboxymethylierende Verbindung in einem großen Überschuß (bezogen auf die Chloressigsäure) eingesetzt werden und so gleichzeitig die Funktion eines Löse- oder Verdünnungsmittels übernehmen.

Als Base wird man üblicherweise Natriumhydroxid als wäßrige, 50%ige Lösung verwenden. Der Einsatz anderer Basen, z. B. Kaliumhydroxid oder auch Mischungen von Kalium- und Natriumhydroxid als wäßrige Lösungen ist gleichfalls ohne Schwierigkeit möglich. Selbstverständlich kann die Reaktion auch kontinuierlich durchgeführt werden.

Der in den folgenden Beispielen benutzt Begriff »Carboxymethylierungsgrad« wird definiert als

$$\frac{\text{Mole erhaltenes Carboxylat} \times 100}{\text{Mole eingesetztes Oxethylat}}.$$

Wenn mit einem Unterschuß von Chloressigsäure, bezogen auf das eingesetzte Oxethylat, gearbeitet wird, um niedrigere Carboxylierungsgrade zu erreichen (vgl. Beispiele 8 und 10) oder aus verfahrenstechnischen Gründen (Beispiel 9), so wird neben dem Carboxymethylierungsgrad auch die Selektivität der Reaktion, bezogen auf die eingesetzte (und in jedem Fall auch vollständig umgesetzte) Chloressigsäure, als Maßstab für die Güte der Umsetzung angegeben.

Der Caboxylatgehalt wird mittels Zweiphasentitration mit Chloroform als Extraktionsmittel und Safranin als Indikator durch Titration mit Cetylpyridiniumchlorid festgestellt.

## Beispiel 1

In einem zylindrischen Rührgefäß von 6,5 l Inhalt und einem Durchmesser von 150 mm werden 2420 g (5 Mol) mit 6 Mol Ethylenoxid oxethylierten Nonylphenols (MARLOPHEN 86 S®) vorgelegt. Das mit einem Doppelmantel versehene Reaktionsgefäß wird über einen Thermostaten auf 70°C aufgeheizt und ein Reaktionsdruck von 12 mbar eingestellt. Der Inhalt wird mit einem gut wirkenden Blattrührer (ca. 700 Upm) gerührt. Am Boden des Reaktors wird mittels einer Zahnradpumpe Produkt aus dem Reaktor entnommen und dieses wieder in den oberen Teil des Reaktors zurückgefahren. Aus diesem Produktkreislauf können ohne Unterbrechung des Vakuums Proben entnommen werden. Über exakt arbeitende Dosierpumpen werden 600 g einer 80%igen wäßrigen Lösung von Monochloressigsäure (5 Mol) (Chloressigsäure mit einem Gehalt von ca. 99%) und 808 g einer 49,5%igen wäßrigen Lösung von Natriumhydroxid (10 Mol) im Verlauf von 3 Stunden getrennt in den Reaktor eingefahren. Man beginnt zweckmäßigerweise mit der Forderung der Lauge etwa 5 bis 10 Minuten früher als mit dem Zufahren der Chloressigsäure; durch diese Maßnahme wird stets eine alkalische Reaktion im Reaktor gewährleistet.

Das eingebrachte und während der Reaktion entstehende Wasser wird über ein ausreichend dimensioniertes Brüdenrohr entfernt, über einen Kühler zum größten Teil kondensiert und der restliche Teil in nachgeschalteten Kühlfallen abgeschieden. Nach einer Reaktionszeit von 60 (120, 180) Minuten hat eine aus dem Reaktor entnommene Probe folgende Analysenwerte (Gewichtsprozent):

| RCOONa*) | NaCl | NaOH | $H_2O$ | RCOONa**) | Zeit (min) |
|---|---|---|---|---|---|
| 34,9 | 3,6 | 0,6 | 0,42 | 36,4 | 60 |
| 64,4 | 7,0 | 0,6 | 0,65 | 72,4 | 120 |
| 87,2 | 9,4 | 0,5 | 1,0 | 97,9 | 180 |
| 88,5 | 9,9 | 0,04 | 0,27 | 98,7 | 200 |

*) Molgewicht 564

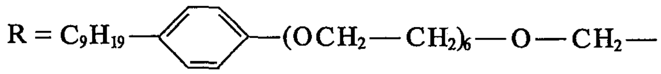

**) berechnet ohne Nebenbestandteile (NaCl, NaOH, $H_2O$).

Während d der anschließenden Nachrührzeit unter Vakuum (20 Minuten) steigt der Carboxylatgehalt im Reaktionsgemisch geringfügig auf 88,5% an. Die insgesamt erhaltene Menge des Reaktionsprodukts beträgt 3137 g. Daraus errechnet sich ein Gehalt von 2776 g carboxymethylierte Verbindung (4,92 Mol). Die eingesetzte Chloressigsäure (5,08 Mol), die vollständig umgesetzt wurde, wird demnach mit einer Selektivität von 98,8% bei der Carboxymethylierungsreaktion umgesetzt, während der Carboxymethylierungsgrad des eingesetzten Oxethylats 98,4% beträgt.

Vergleichsbeispiel 1

Beispiel 1 wird wiederholt, jedoch der Reatkionsdruck auf 65 mbar und die Reaktionstemperatur auf 75°C eingestellt. Im Reaktionsgemisch stellt sich ein mittlerer Wassergehalt von 1,7 Gewichtsprozent ein. Es wird ein Gehalt von 78,2 Gewichtsprozent carboxymethyliertem Produkt (2543 g) erreicht, was einem Carboxymethylierungsgrad von 87% entspricht.

Vergleichsbeispiel 1a

In diesem Beispiel werden Druck- und Temperaturbedingungen und mithin auch der Wassergehalt der japanischen Anmeldung Sho-50-24 215 eingehalten.

Das Vergleichsbeispiel 1 wird wiederholt, jedoch werden 2420 g (5 Mol) oxethyliertes Nonylphenol (mit 6 Ethylenoxid) vorgelegt und mit 595 g Natriumchloracetat (5 Mol, 98%ig) vermischt. Sodann werden unter Rühren bei einer Temperatur von 75°C und einem Druck von 65 mbar 407 g Natronlauge (5 Mol, 49,2%ig) in die Reaktionsmischung mittels einer Dosierpumpe im Verlauf von drei Stunden zugefahren. Es stellt sich ein Wassergehalt zwischen 1,5 bis 1,7 Gewichtsprozent im Reaktionsgemisch ein. Nach Beendigung der Zufuhr der Natronlauge wird noch 20 Minuten unter Beibehaltung des Reaktionsdrucks von 65 mbar weitergeführt und nach Aufheben des Vakuums nach weiteren 10 Minuten das Reaktionsprodukt analysiert. Es wird ein Carboxymethylatgehalt von nur 58,5% gefunden (1835 g ≙ 3,25 Mol), woraus sich ein Carboxymethylierungsgrad von 65% errechnet.

Beispiel 1, Vergleichsbeispiel 1 und 1a zeigen, daß der Wassergehalt im Reaktionsgemisch $<1,25$ Gewichtsprozent sein muß, wenn eine nahezu quantitative Carboxymethylierung des Oxethylats erreicht werden soll, und daß die gleichzeitige Zugabe von wäßriger Natronlauge und wäßriger Chloressigsäure den Umsetzungsgrad erheblich verbessert.

Beispiele 2 bis 6

In den folgenden Beispielen 2 bis 6 wird wie in Beispiel 1 gearbeitet, jedoch bei einer Reaktionstemperatur von 90°C. Der Reaktionsdruck wird variiert und damit im Reaktionsgemisch ein unterschiedlicher Wassergehalt von 0,4 bis 1,5 Gewichtsprozent eingestellt. Hierbei ergibt sich, daß bei einem Wassergehalt von 0,4 bis 0,8 Gewichtsprozent ein Carboxymethylierungsgrad von 95 bis 99% erreicht wird (Beispiele 2, 3, 4), bei einem Wassergehalt von 1,3 Gewichtsprozent werden noch ca. 90% (Beispiel 5) erreicht und bei einem noch höheren Wassergehalt (Beispiel 6) fällt der Carboxymethylierungsgrad unter 90% ab. In Beispiel 3a wurde die Eintropfzeit der Chloressigsäure und der Natronlauge, die bei allen übrigen Beispielen drei Stunden betrug, auf 1,5 Stunden verringert, was ohne Einbuße bezüglich des zu erzielenden Carboxymethylierungsgrades möglich ist (vgl. Beispiele 3 und 3a).

Tabelle I

| | Beispiel 2 | 3 | 3a*) | 1 | 4 | 5 | 6 |
|---|---|---|---|---|---|---|---|
| | | | | | | nicht erfindungsgemäß | |
| Druck, mbar | 12 | 25 | 25 | 12 | 50 | 75 | 100 |
| Temp., °C | 90 | 90 | 90 | 70 | 90 | 90 | 90 |
| Wassergehalt, Gew.-% | 0,45 | 0,58 | 0,55 | 0,65 | 0,80 | 1,30 | 1,52 |
| RCOONa, Gew.-% im Endprodukt | 89,4 | 88,2 | 88 | 88,5 | 85,6 | 80,6 | 78,6 |
| Carboxymethylierungsgrad, % | 99,4 | 98,1 | 97,9 | 98,4 | 95,2 | 89,7 | 87,4 |

*) Eintropfzeit der Lauge und der Chloressigsäure 1,5 Stunden, bei allen übrigen 3 Stunden.

R = 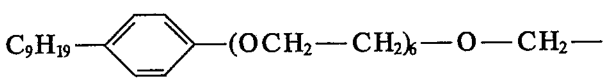

## Beispiel 7

In der in Beispiel 1 beschriebenen Apparatur wurden 2420 g mit 6 Mol Ethylenoxid oxethyliertes Nonylphenol (5 Mol) vorgelegt und in der beschriebenen Weise 600 g einer 80%igen wäßrigen Lösung von Chloressigsäure (5 Mol) sowie eine Mischung, bestehend aus 404 g einer 49,5%igen wäßrigen Natronlauge und 561 g einer 50%igen wäßrigen Kalilauge (jeweils 5 Mol von beiden Basen) bei einem Reaktionsdruck von 12 mbar und einer Reaktionstemperatur von 80°C innerhalb von drei Stunden zugefahren. Während der Zufuhr der wäßrigen Lauge und der wäßrigen Chloressigsäure stellt sich im Reaktionsgemisch ein Wassergehalt von 0,57 Gewichtsprozent ein. Das helle Endprodukt wird nach Beendigung der Zugabe der Reaktionskomponenten noch 30 Minuten bei der Reaktionstemperatur gerührt.

| | |
|---|---|
| RCOONa/K | 88,1 Gewichtsprozent |
| NaCl/KCl | 10,8 Gewichtsprozent |
| NaOH | 0,01 Gewichtsprozent |
| $H_2O$ | 0,20 Gewichtsprozent |

R = 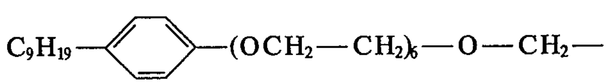

Aus den Analysenwerten des Endproduktes (3193 g) errechnet sich ein Gehalt von 4,92 Mol Na/K-Carboxylatgemisch (MG 572), d. h. der Carboxymethylierungsgrad beträgt 98,4%.

## Beispiel 8

In einem 100-l-Rührkessel werden 23,79 kg eines mit 6,1 Mol Ethylenoxid/Mol oxethylierten Nonylphenols vorgelegt (48,71 Mol). Der Kessel wird auf eine Temperatur von 90°C aufgeheizt und auf einen Druck von 18 bis 20 bar evakuiert. Unter starkem Rühren werden über getrennte Zuleitungen, die in dem vorgelegten Nonylphenoloxethylat abgetaucht sind, 4,26 kg Chloressigsäure, Gehalt 99%ig, als ca. 80%ige wäßrige Lösung (44,6 Mol) und 7,3 kg einer wäßrigen 49%igen Natronlauge (89,4 Mol) analog Beispiel 1 innerhalb von drei Stunden zugefahren.

Das Wasser wird über eine ausreichend dimensionierte beheizte Brüdenleitung (∅ 70 mm) aus dem Kessel entfernt und über Kühler kondensiert. Insgesamt werden 7,07 kg Wasser kondensiert. Der Wassergehalt im Reaktionsgemisch während des Zudosierens der Lauge und der Chloressigsäure schwankt zwischen 0,6 bis 0,7 Gewichtsprozent. Nach der erfolgten Zugabe der Chloressigsäurelösung und der Natronlauge wird das Reaktionsprodukt noch 20 Minuten unter den angegebenen Reaktionsbedingungen gerührt und dann in warmem Zustand aus dem Kessel abgelassen.

Das Endprodukt (30,01 kg) besitzt die folgenden Analysenwerte:

| | |
|---|---|
| RCOONa (MG 568) | 81,6 Gewichtsprozent |
| NaCl | 9,2 Gewichtsprozent |
| NaOH | 0,04 Gewichtsprozent |
| $H_2O$ | 0,26 Gewichtsprozent |

R = $C_9H_{19}$— 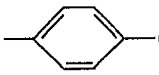 —$(OCH_2—CH_2)_{6.1}$—O—$CH_2$—

Daraus errechnet sich ein Carboxymethylierungsgrad von 88,7%. Da das Molverhältnis von Chloressigsäure zu Oxethylat = 44,6/48,71 entsprechend 0,916 ist, wird die eingesetzte Chloressigsäure mit einer Selektivität von 96,9% für die Carboxymethylierungsreaktion verbraucht.

## Beispiel 9

2568 g $C_{14}$-Alkohol (12 Mol) (ALFOL 14 RD®) werden mit 670 g Monochloressigsäure (99%ig = 7 Mol) gelöst in 167 g Wasser sowie 1135 g einer 49,3%igen wäßrigen Natronlauge (14 Mol) im Verlauf von 4 Stunden bei einer Temperatur von 95°C und einem Druck von 25 mbar in der in Beispiel 1 beschriebenen Apparatur umgesetzt. Im Reaktionsgemisch stellt sich hierbei ein Wassergehalt von 0,65 Gewichtsprozent ein. Von dem im Überschuß vorhandenen Alkohol werden zusammen mit dem Wasserdampf 98 g während der Reaktion abdestilliert.

Nachdem die Zugabe der Lauge und der Chloressigsäure beendet ist, wird noch eine Stunde bei Normaldruck weitergerührt.

Das so erhaltene helle Reaktionsprodukt (3447 g) enthält 54,3 Gewichtsprozent des carboxylmethylierten Alkohols und 0,2 Gewichtsprozent Wasser. Daraus errechnet sich eine Menge von 1872 g (6,37 Mol) Carboxylat (MG 294). Bezogen auf die eingesetzten 7 Mol Chloressigsäure wird diese mit einer Selektivität von 91% umgesetzt.

## Beispiel 10

2064 g $C_{12/18}$-Alkohol mit 3 Mol EO oxethyliert (6 Mol) werden in der in Beispiel 1 beschriebenen Apparatur vorgelegt. 4,8 Mol (458 g) einer 99%igen Chloressigsäure werden in 1150 g Wasser gelöst und diese Lösung analog Beispiel 1 mit 781 g 49,2%iger Natronlauge (9,6 Mol) im Verlauf von 3 Stunden unter Rühren bei einem Druck von 26 mbar und einer Temperatur von ca. 82°C in das Oxethylat eindosiert. Eine nach einer Reaktionszeit von 1,5 Stunden analysierte Probe ergibt einen Wassergehalt von 0,74 Gewichtsprozent im Reaktionsgemisch.

Nachdem die Zugabe der Lauge und der Chloressigsäurelösung beendet ist, wird noch 20 Minuten unter Reaktionsbedingungen zur Vervollständigung der Reaktion weitergeführt. Das Endprodukt (2690 g) hat die folgenden Analysenwerte:

| | |
|---|---|
| RCOONa (MG 424) | 70,1 Gewichtsprozent |
| NaCl | 10,4 Gewichtsprozent |
| NaOH | 0,43 Gewichtsprozent |
| Wasser | 0,62 Gewichtsprozent |

R = $C_{12/18}—(O—CH_2—CH_2)_3—O—CH_2—$

Daraus errechnet sich ein Carboxymethylierungsgrad von 74,1%. Die eingesetzte Chloressigsäure wird mit einer Selektivität von 92,6% umgesetzt. Aufgrund des gewählten Molverhältnisses von 1 : 0,8 könnte höchstens ein Carboxymethylierungsgrad von 80% erreicht werden.

## Beispiel 11

2986 g Dinonylphenol mit 9,1 Mol Ethylenoxid oxethyliert (4 Mol) werden in der in Beispiel 1 beschriebenen Apparatur vorgelegt. Im Verlauf von 2 Stunden werden 478 g einer 80%igen wäßrigen Chloressigsäure (4 Mol) und 647 g einer 49,5%igen wäßrigen Natronlauge (8 Mol) analog wie in Beispiel 1 beschrieben in das Reaktionsgefäß eingefahren. Hierbei wird ein Druck von 22 bis 25 mbar und eine Temperatur von ca. 90°C eingestellt. Die Drehzahl des Rührers beträgt ca. 700 Upm. Der Wassergehalt, der sich im Reaktionsgemisch einstellt, erreicht einen Wert von 0,5 Gewichtsprozent. Nachdem die Zugabe der Lauge und der Chloressigsäure beendet ist, rührt man noch 20 Minuten weiter bei

einem Druck von 22 mbar und bei einer Temperatur von 90° C. Das gelbliche, bei Raumtemperatur noch fließfähige Endprodukt (3558 g) hat die folgenden Analysenwerte:

| | |
|---|---|
| RCOONa (MG 827) | 84,0 Gewichtsprozent |
| NaCl | 7,3 Gewichtsprozent |
| NaOH | 0,02 Gewichtsprozent |
| $H_2O$ | 0,51 Gewichtsprozent |

R = 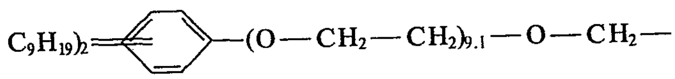

Daraus errechnet sich eine Menge von 2988 g (3,61 Mol) carboxymethylierter Verbindung und ein Carboxymethylierungsgrad von 90,3%.

Beispiel 12

In der Apparatur gemäß Beispiel 1 werden 1508 g (3 Mol) MARLOPHEN 814® (Nonylphenol mit 14 Mol EO oxethyliert, MG 836) vorgelegt. Bei einem Druck von 25 mbar und bei einer Temperatur von 90° C werden 359 g Chloressigsäure (80%ige wäßrige Lösung, 3 Mol) und 488 g einer 48,2%igen Natronlauge (6 Mol) im Verlauf von 1,5 Stunden unter Rühren zugefahren. Der Wassergehalt im Reaktionsgemsich beträgt 0,58 Gewichtsprozent. Nach Beendigung der Zugabe der Natronlauge und der Chloressigsäure wird noch 20 Minuten bei unveränderten Reaktionsbedingungen weitergerührt. Das anfallende Endprodukt (2939 g) besitzt folgende Analysenwerte:

| | |
|---|---|
| RCOONa (MG 916) | 87,7 Gewichtsprozent |
| NaCl | 6,4 Gewichtsprozent |
| NaOH | 0,03 Gewichtsprozent |
| $H_2O$ | 0,5 Gewichtsprozent |

R = 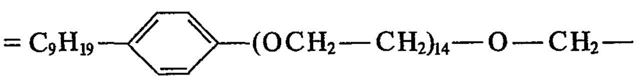

Aus den angegebenen Werten errechnet sich ein Carboxymethylierungsgrad von 93,8%.

Beispiel 13

2500 g (6,61 Mol) Laurylmercaptan, das mit 4 Mol Ethylenoxid oxethyliert wird, wird mit 583 g (6,1 Mol) Chloressigsäure (Gehalt 99%ig), die in Wasser gelöst als 80%ige wäßrige Lösung vorliegt und mit 985 g (12,2 Mol) einer wäßrigen, 49%igen Natronlauge wie in Beispiel 1 beschrieben, umgesetzt. Die Reaktionstemperatur beträgt 75° C, der Reaktionsdruck 12 mbar. Während des Zudosierens der Komponenten stellt sich in Reaktionsgefäß ein Wassergehalt von 0,62 Gewichtsprozent ein. Nach 3 Stunden ist die Zufuhr beendet; anschließend wird noch 20 Minuten unter den Reaktionsbedingungen weitergerührt. Im Reaktionsgefäß verbleiben 3368 g Endprodukt mit folgenden Analysenwerten:

| | |
|---|---|
| RCOONa (MG 458) | 78,1 Gewichtsprozent |
| NaCl | 12,1 Gewichtsprozent |
| NaOH | 0,01 Gewichtsprozent |
| $H_2O$ | 0,53 Gewichtsprozent |

$R = C_{12}H_{25}-S-(CH_2-CH_2-O)_4-CH_2-$

Daraus errechnet sich eine Menge von 2630 g carboxymethylierter Verbindung (5,74 Mol). Daraus ergibt sich ein Carboxymethylierungsgrad von 86,8%. Da das Molverhältnis der eingesetzten Verbindungen lediglich 1 : 0,923 ist, hätte nur ein Carboxymethylierungsgrad von maximal 92,3% erreicht werden können, somit entspricht der gefundene Carboxymethylierungsgrad von 86,8% einer Selektivität von

$$\frac{5{,}74 \times 100}{6{,}10} = 94{,}1\%,$$

bezogen auf die umgesetzte Chloressigsäure.

## Patentansprüche

1. Verfahren zur Herstellung von carboxymethylierten Alkoholen, Etheralkoholen, Thiolalkoholen oder Alkylphenolen durch Umsetzung zwischen (Thio-)Etheralkoholen oder Alkylphenolen mit einem Salz der Chloressigsäure und Alkalihydroxid, dadurch gekennzeichnet, daß eine wäßrige Lösung von höchstens der einfachen molaren Menge freier Monochloressigsäure und die zweifache molare Menge einer wäßrigen Base (bezogen auf Alkohol oder Phenol), getrennt aber gleichzeitig unter Rühren bei erhöhter Temperatur und vermindertem Druck einem vorgelegten gesättigten oder ungesättigten, geradkettigen oder verzweigten Alkohol oder Thiolalkohol mit 8 bis 20 C-Atomen oder einem Monoalkyl- oder Dialkylphenol mit 7 bis 18 C-Atomen in der Alkylkette oder einem Etheralkohol der Formel

$$R-(OCH_2-CH_2)_n-OH$$

bzw. einem Thioetheralkohol der Formel

$$R-S-(CH_2-CH_2-O)_n-H,$$

wobei R einen gesättigten oder ungesättigten, geradkettigen oder verzweigten Alkylrest mit 8 bis 20 C-Atomen oder beim Etheralkohol auch einen Monoalkyl- oder Dialkylphenylrest mit 7 bis 18 C-Atomen in der Alkylkette und n eine ganze Zahl von 1 bis 30 darstellt, zugegeben werden, und während der Zugabe der Reaktionskomponenten der Wassergehalt im Reaktionsgemisch 0,3 bis 1,25 Gewichtsprozent beträgt, wobei mit der Zufuhr der Base 5 bis 10 Minuten vor der Zufuhr der Chloressigsäure begonnen wird.

2. Verfahren nach Anspruch 1, dadurch gekennzeichnet, daß während der gesamten Reaktion der Wassergehalt im Reaktionsgemisch 0,3 bis 0,8 Gewichtsprozent beträgt.

## Claims

1. A process for the production of a carboxymethylated alcohol, etheralcohol, thioalcohol or alkylphenol by reaction between a (thio-)alcohol, (thio-)etheralcohol or alkylphenol and a salt of chloroacetic acid and an alkali metal hydroxide, characterised in that an aqueous solution containing at most one molar quantity of free monochloroacetic acid and two molar quantities of an aqueous base (based on the molar quantity of alcohol or phenol) are separately but simultaneously added with stirring and at elevated temperature and reduced pressure to an already present, saturated or unsaturated, linear or branched alcohol or thioalcohol of 8 to 20 carbon atoms or monoalkyl or dialkyl phenol of 7 to 18 carbon atoms in the alkyl moiety or etheralcohol of the formula

$$R-(OCH_2-CH_2)_n-OH$$

and/or thioetheralcohol of the formula

$$R-S-(CH_2-CH_2-O)_n-H,$$

where R is a saturated or unsaturated, linear or branched alkyl radial of 8 to 20 carbon atoms or in the case of an etheralcohol can alternatively be a monoalkyl- or dialkyl-phenyl radical of 7 to 18 carbon atoms in the alkyl moiety and n is an integer of 1 to 30, and in that the water content of the reaction mixture during the addition of the reaction components amounts to 0.3 to 1.25% by weight, the addition of the base commencing about 5 to 10 minutes before the addition of the chloroacetic acid.

2. A process according to claim 1, characterised in that the water content of the reaction mixture amounts to 0.3 to 0.8% by weight throughout the entire reaction.

## Revendications

1. Procédé de préparation d'alcools carboxyméthylés, d'éthers-alcools, de thio-alcools ou d'alkyl-phénols par réaction de (thio-)alcools, de (thio-)éthers-alcools ou d'alkyl-phénols sur un sel de l'acide chloracétique et un hydroxyde alcalin, caractérisé par le fait que l'on ajoute séparément mais simultanément avec agitation, à température élevée et à pression réduite, une solution aqueuse d'au maximum une fois la quantité molaire d'acide monochloracétique libre et deux fois la quantité molaire d'une base aqueuse (relativement à l'alcool ou au phénol), à un alcool ou thio-alcool saturé ou insaturé, à chaîne ou ramifié, placé initialement, contenant de 8 à 20 atomes de carbone, ou à un mono-alkyl- ou dialkyl-phénol contenant de 7 à 18 atomes de carbone dans la chaîne alkyle ou à un éther-alcool de la formule

$$R-(OCH_2-CH_2)_n-OH$$

ou à un thio-éther-alcool de la formule

$$R-S-(CH_2-CH_2-O)_n-H,$$

dans lesquelles R représente un radical alkyle saturé ou insaturé, à chaîne droite ou ramifié, contenant de 8 à 20 atomes de carbone ou encore, dans le cas de l'éther-alcool, également un radical mono-alkyl- ou dialkyl-phényle contenant de 7 à 18 atomes de carbone dans la chaîne alkyle, et n représente un nombre entier d'une valeur de 1 à 30, et que, pendant l'addition des constituantes de réaction, la teneur en eau du mélange réactionnel est de 0,3 à 1,25% en poids, l'amenée de la base commençant environ 5 à 10 minutes avant l'amenée de l'acide chloracétique.

2. Procédé selon la revendication 1, caractérisé par le fait que pendant toute la réaction, la teneur en eau du mélange réactionnel est de 0,3 à 0,8% en poids.